(19) Europäisches Patentamt
European Patent Office
Office européen des brevets

(11) Veröffentlichungsnummer: **0 244 632 B1**

# (12) EUROPÄISCHE PATENTSCHRIFT

(45) Veröffentlichungstag der Patentschrift: **20.02.91**

(51) Int. Cl.$^5$: **B01J 8/06**, C07C 45/39

(21) Anmeldenummer: **87104722.1**

(22) Anmeldetag: **31.03.87**

Die Akte enthält technische Angaben, die nach dem Eingang der Anmeldung eingereicht wurden und die nicht in dieser Patentschrift enthalten sind.

## (54) Rohrbündelreaktor, dessen Verwendung bei exothermen organischen Reaktionen und Verfahren zur Herstellung von Ketonen und Aldehyden.

(30) Priorität: **11.04.86 DE 3612213**

(43) Veröffentlichungstag der Anmeldung:
**11.11.87 Patentblatt 87/46**

(45) Bekanntmachung des Hinweises auf die Patenterteilung:
**20.02.91 Patentblatt 91/08**

(84) Benannte Vertragsstaaten:
**CH DE ES FR GB IT LI SE**

(56) Entgegenhaltungen:
**DE-B- 1 222 032**
**US-A- 3 482 948**
**US-A- 4 154 762**
**US-A- 4 165 342**

(73) Patentinhaber: **BASF Aktiengesellschaft**
**Carl-Bosch-Strasse 38**
**D-6700 Ludwigshafen(DE)**

(72) Erfinder: **Brenner, Karl**
**Riedsaumstrasse 40**
**D-6700 Ludwigshafen(DE)**
Erfinder: **Ruppel, Wilhelm, Dr.**
**Koenigsberger Strasse 13**
**D-6830 Schwetzingen(DE)**
Erfinder: **Woerz, Otto, Dr.**
**Friedrich-Pietzsch-Strasse 10**
**D-6701 Friedelsheim(DE)**
Erfinder: **Halbritter, Klaus, Dr.**
**Schwarzwaldstrasse 19**
**D-6800 Mannheim 1(DE)**
Erfinder: **Scheiper, Hans-Juergen**
**Pfalzring 46**
**D-6704 Mutterstadt(DE)**
Erfinder: **Aquila, Werner, Dr.**
**Meissener Weg 35**
**D-6800 Mannheim 31(DE)**
Erfinder: **Hartwig, Fuchs**
**An der Froschlache 23**
**D-6700 Ludwigshafen(DE)**

**EP 0 244 632 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft einen Rohrbündelreaktor zur Durchführung organischer Reaktionen in der Gasphase mit zwischen Rohrböden angeordneten Reaktionsrohren. Die Erfindung betrifft ferner die Verwendung des Rohrbündelreaktors bei exothermen organischen Reaktionen. Gegenstand der Erfindung ist auch ein Verfahren zur Herstellung aliphatischer, aromatischer oder araliphatischer Ketone und Aldehyde unter Verwendung des erfindungsgemäßen Rohrbündelreaktors. Der erfindungsgemäße Rohrbündelreaktor ist besonders geeignet zur Durchführung katalytisch beschleunigter Reaktionen, die stark exotherm sind und deren Reaktionsgeschwindigkeit stark von der Temperatur abhängt.

Wenn die Stabilität von Reaktanden und Produkten dies gestattet, kann man derartige Reaktionen adiabatisch bei extrem kurzen Verweilzeiten durchführen. Beispiele sind die Ammonoxidation an Platinnetzen oder die oxidative Dehydrierung von Methanol zu Formaldehyd an einer Schüttung von Silberkristallen von ca. 20 mm Höhe.

Sind die Reaktanden oder Produkte instabil, so daß eine adiabatische Reaktionsführung nicht möglich ist, so muß über eine Wärmetauschfläche zumindest ein Teil der Reaktionswärme abgeführt werden. Man kann den Katalysator entweder zwischen (Linde-Reaktor: DE-A-34 14 717) oder in (Rohrbündelreaktor: Chemie Ingenieur Technik 51 (79) S. 257-265) die Rohre eines Wärmetauschers einbringen. Reaktoren dieser Art besitzen Rohre von mehreren Zentimetern Durchmesser und Längen von 2 bis 20 Metern, d.h. sie haben relativ lange Verweilzeiten. Bei stark exothermen Reaktionen bildet sich ein "hot-spot" aus, der häufig zu erheblichen Selektivitätsverlusten oder Schädigungen des Kontaktes führt. Man kann versuchen, den "hot-spot" dadurch zu beseitigen, daß der Katalysator am Beginn der Schüttung mit inertem Material verdünnt wird, oder daß man bei partiellem Umsatz fährt und Kreisgas in den Reaktor zurückführt. Dies erzwingt einen großen technischen Aufwand, nämlich große Reaktoren, eine umfangreiche Abgasaufarbeitung und die Bewältigung großer Kreisgasmengen, und dennoch sind häufig Selektivitätsverluste nicht zu vermeiden.

Wenn zur thermischen Instabilität von Reaktanden und Produkten eine große Reaktionswärme und eine hohe Aktivierungsenergie hinzukommen, so ist ein Reaktor erforderlich, der einerseits die sehr kleine Verweilzeit des adiabatischen Reaktors mit einer kurzen Katalysatorschüttung und andererseits mindestens die gute Wärmeabfuhr des Rohrbündelreaktors vereinigt.

Die EP-A-55 354 beschreibt ein katalytisches Verfahren zur kontinuierlichen Herstellung von 3-Alkyl-buten-1-alen durch oxidative Dehydrierung der entsprechenden Alkohole in einem Rohrreaktor. Der Rohrreaktor ist ersichtlich von üblicher Bauart, über Rohrabmessungen und sonstige konstruktive Ausgestaltungen ist in der Druckschrift nichts gesagt, da es nach der bekannten Lehre darauf auch offensichtlich nicht ankommt.

Der Erfindung liegt die Aufgabe zugrunde, einen Rohrbündelreaktor der eingangs genannten Art so auszugestalten, daß bei stark exothermen Reaktionen, insbesondere an extrem aktiven Katalysatoren, beispielsweise an Edelmetallkontakten, in der Gasphase eine wohldefinierte Reaktionsführung möglich ist. Insbesondere sollen bei derartigen Reaktionen hohe Selektivitäten erzielt werden.

Diese Aufgabe wird gelöst mit einem Rohrbündelreaktor der eingangs genannten Art, der dadurch gekennzeichnet ist, daß die Reaktionsrohre einen Innendurchmesser im Bereich von 0,5 bis 3 cm aufweisen, das Verhältnis Reaktionsrohrlänge zu Innendurchmesser zwischen 2 und 10 liegt und daß die Reaktionsrohre von einem fluiden Wärmeträger in seitlicher Richtung umströmt werden.

Nach einer bevorzugten Ausführungsform weisen die Reaktionsrohre eine Länge von 5 bis 20 cm auf.

Besonders bevorzugt ist ein Rohrinnendurchmesser von 1 bis 2 cm.

Als fluider Wärmeträger kommen die üblichen Wärmeträger in Betracht, besonders bevorzugt sind Salzschmelzen. Die Strömungsrichtung des fluiden Wärmeträgers verläuft im wesentlichen senkrecht zur Längsachse des jeweiligen Reaktionsrohrs.

Mit Hilfe des erfindungsgemäßen Kurzrohrbündelreaktors läßt sich eine extrem hohe und gleichmäßige Anströmung der Rohre und damit eine besonders große Wärmeabfuhr erzielen. Die kompakte Bauweise erlaubt zudem eine einfache Führung des Wärmeträgers und führt zu einem preiswerten Reaktor mit hoher Raum-Zeit-Ausbeute. Darüber hinaus müssen über die Bedürfnisse des speziellen chemischen Prozesses hinausgehende Inertgasmengen nicht oder nur in geringfügigem Maße zugefahren werden.

Während bei den bekannten Rohrbündelreaktoren das Verhältnis Rohrlänge zu Rohrdurchmesser bei 100 bis 1000 liegt, liegt dieses Verhältnis beim erfindungsgemäßen Kurzrohrbündelreaktor im Bereich von 2 bis 10. Durch die kurzen Rohre erhält ein technischer Reaktor die Form einer flachen Scheibe mit relativ kleinem Volumen. Bei dieser Reaktorform braucht der Wärmeträger nicht in einem großen, dreidimensionalen Raum verteilt werden.

Gegenstand der Erfindung ist auch die Verwendung des erfindungsgemäßen Rohrbündelreaktors bei

exothermen organischen Reaktionen. Insbesondere ist weiterer Gegenstand der Erfindung ein Verfahren zur Herstellung aliphatischer, aromatischer oder araliphatischer Ketone und Aldehyde durch oxidative Dehydrierung der entsprechenden Alkohole mit einem Katalysator der Gruppe 1b des Periodensystems der Elemente. Dieses Verfahren ist dadurch gekennzeichnet, daß man die oxidative Dehydrierung in einem wie zuvor definierten Rohrbündelreaktor durchführt.

Eine besonders vorteilhafte Ausgestaltung des erfindungsgemäßen Verfahrens dient zur Herstellung von 3-Alkyl-buten-1-alen der allgemeinen Formel I

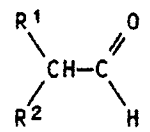

worin $R^1$ für Wasserstoff steht und $R^2$ den Rest

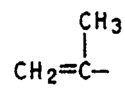

bedeutet oder $R^1$ und $R^2$ zusammen den Rest

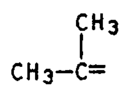

bedeuten, das dadurch gekennzeichnet ist, daß man 3-Alkyl-buten-1-ole der Allgemeinen Formel II

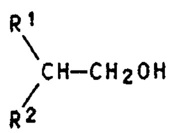

worin $R^1$ und $R^2$ wie zuvor definiert sind, mit Sauerstoff bei Temperaturen von 300 bis 600 °C über den Katalysator leitet und die Reaktionsgase nach dem Abkühlen in üblicher Weise aufarbeitet.

Unter Verwendung des erfindungsgemäßen Rohrbündelreaktors erhält man die gewünschten Wertprodukte mit hohen Selektivitäten und Umsätzen.

Die Erfindung und der Stand der Technik sind in den Figuren 1 bis 7 der Zeichnungen näher erläutert. In der Zeichnung bedeuten:

Figur 1 einen Querschnitt durch einen schematisch dargestellten Längsstromreaktor des Standes der Technik;

Figur 2 einen Querschnitt durch einen schematisch dargestellten Radialstromreaktor des Standes der Technik;

Figur 3 einen Querschnitt durch eine schematische Darstellung eines erfindungsgemäßen Kurzrohrreaktors;

Figur 4 einen Ausschnitt aus dem Kurzrohrreaktor der Figur 3;

Figur 5 eine Aufsicht auf einen Teil des Kurzrohrreaktors gemäß Figur 4;

Figur 6 eine Anlage zur Durchführung exothermer organischer Reaktionen, insbesondere zur oxidativen Dehydrierung von Ketonen und Aldehyden unter Verwendung eines erfindungsgemäßen Rohrbündelreaktors; und

Figur 7 eine nicht erfindungsgemäße Anlage zur Durchführung derselben Reaktion mit einen Tiegelreaktor (Vergleichsbeispiele 1 bis 4).

Beim Längsstromreaktor des Standes der Technik gemäß Figur 1 werden die Reaktionsrohre parallel zur Längsachse des jeweiligen Reaktionsrohrs gleichmäßig mit einem Salzstrom angeströmt. Nachteilig an dem Längsstromreaktor ist, daß der salzseitige Wärmeübergangskoeffizient infolge der Längsanströmung relativ gering ist.

Die Queranströmung der Rohre beim Radialstromreaktor gemäß Figur 2 führt zwar zu einem deutlich höheren Wärmeübergangskoeffizienten auf der Salzseite, jedoch sind Umlenkungen des Salzstromes erforderlich. Um eine ungleichmäßige Anströmung der Rohre zu vermeiden, kann nur ein Ringraum mit Reaktionsrohren bestückt werden.

Der in Figur 3 schematisch dargestellte erfindungsgemäße Kurzrohrreaktor weist eine Reihe von Reaktionsrohren A auf, die zwischen 2 Rohrböden B befestigt sind. Zwischen den Rohrböden B strömt in einer zur Reaktionsrohrlängsachse im wesentlichen senkrechten Richtung ein Wärmeträger und umströmt die Reaktionsrohre A. Die Strömungsrichtung des Wärmeträgers, bei dem es sich vorzugsweise um eine Salzschmelze handelt, ist in der Figur 3 durch die nach links weisenden Pfeile angedeutet. Der Reaktionsstrom ist durch den nach unten deutenden Pfeil bezeichnet. Der Reaktionsstrom erfolgt durch die Reaktionsrohre. Die Reaktionsrohre sind zweckmäßig zueinander versetzt angeordnet.

In der Figur 4 ist eine Schnittansicht durch einen erfindungsgemäßen Kurzrohrreaktor teilweise dargestellt. Ebenso wie in der Figur 3 sind in der Figur 4 die Reaktionsrohre mit A und die Rohrböden mit B bezeichnet. Die in der Figur 4 dargestellte Ausführungsform weist einen Reaktionsrohrinnendurchmesser von vorzugsweise 10 bis 15 mm und eine Rohrlänge von vorzugsweise 50 bis 200 mm auf. Die Rohre werden mit Hilfe von Automaten in die Rohrböden geschweißt. In der Figure 5 ist ein Rohrboden in der Aufsicht zu sehen. Der Rohrboden B ist mit Öffnungen versehen, in welche die Reaktionsrohre eingesetzt sind. Es ist ersichtlich, daß die Konstruktion des erfindungsgemäßen Kurzrohrreaktors sehr einfach ist. Das wirkt sich in den Fertigungskosten aus. Insbesondere bei druckloser Ausführung läßt sich der Kurzrohrreaktor mit geringem Materialaufwand besonders kostengünstig fertigen.

Die Reaktionsrohre werden vom Wärmeträger, vorzugsweise einer Salzschmelze, in Querrichtung angeströmt. Hierdurch ergibt sich über die Rohrlänge eine gleichmäßige Anströmung. Infolge der sehr hohen spezifischen Kühlfläche läßt sich die Temperaturverteilung im Reaktionsrohr mit Hilfe der Salztemperatur definiert einstellen. Man kann somit bei variabler Zusammensetzung des Reaktorzulaufes stets eine optimale Ausbeute an Wertprodukt erzielen. Bei den vorzugsweise eingesetzten Edelmetallkatalysatoren kann der Einsatz von Inertgasen stark reduziert werden.

Im Vergleich zum Stand der Technik ergeben sich beim erfindungsgemäßen Rohrbündelreaktor insbesondere die folgenden Vorteile:

1. Stark exotherme organische Reaktionen, vorzugsweise an Edelmetallkontakten, können bei definierten Temperaturen durchgeführt werden. Eine flexible Anpassung der Reaktionsbedingungen an die jeweiligen Reaktanden ist möglich.
2. Der erfindungsgemäße Kurzrohrreaktor erlaubt eine sehr gleichmäßige Queranströmung aller Rohre. Hieraus resultiert eine sehr kompakte Bauweise mit maximalen Wärmeübergangskoeffizienten.
3. Selbst bei Verwendung von feinkörnigem Katalysatormaterial in den Reaktionsrohren ist der Druckabfall in den Rohren gering. Verwendet man poröse Katalysatoren, ergeben sich auf einfache Weise kurze Diffusionswege in den Poren des Katalysators.

Der erfindungsgemäße Kurzrohrreaktor eignet sich insbesondere zur kontinuierlichen Durchführung von katalytisch beschleunigten Reaktionen, die stark exotherm sind und deren Reaktionsgeschwindigkeit stark von der Temperatur abhängt. Als solche Reaktionen kommen beispielsweise in Frage: Oxidationen, insbesondere oxidative Dehydrierungen und Halogenierungen.

Der Kurzrohrreaktor eignet sich insbesondere zur Herstellung von aliphatischen, aromatischen und araliphatischen Ketonen und Aldehyden durch oxidative Dehydrierung von entsprechenden Alkoholen in Gegenwart eines Katalysators der Gruppe 1b des Periodensystems der Elemente.

Als Ausgangsprodukte kommen Alkohole der allgemeinen Formel II'

$$R^1-\underset{\displaystyle R^2}{\underset{|}{CH}}-OH \qquad (II')$$

in Betracht,
wobei $R^1$ und $R^2$ gleich oder verschieden sind und für Wasserstoff oder einen gesättigten oder ungesättigten, verzweigten oder unverzweigten aliphatischen Rest, vorzugsweise mit 1 bis 20, insbesondere 1 bis 12 Kohlenstoffatomen, darüber hinaus für einen ein- oder mehrfach substituierten Aryl-, vorzugsweise Phenylrest, der, wenn er substituiert ist, vorzugsweise 1- oder 2-mal, insbesondere durch $C_1$- bis $C_5$-Alkylreste und $C_1$-bis $C_5$-Alkoxyreste substituiert ist, oder wobei $R^1$ und $R^2$ auch für einen Aralkylrest stehen können.

Es kommen z.B. als Ausgangsstoffe der allgemeinen Formel II' in Frage: Methanol, Ethanol, Propanol, iso-Propanol, n-Butanol, iso-Butanol, sek.Butanol, n-Pentanol, 2-Pentanol, 3-Pentanol, 2-Methylbutanol, 3-Methylbutanol, Hexanol, 2-Hexanol, 3-Hexanol, 2-Methylhexanol, 3-Methylhexanol, 2-Ethylhexanol, Heptanol, 2-Heptanol, But-2-en-1-ol, But-3-en-1-ol, 2-Methyl-but-3-en-1-ol, 3-Methyl-but-3-en-1-ol, Pent-3-en-1-ol, Hex-2-en-1-ol, Octen-1-en-3-ol, 2-Methylbenzylalkohol, 3-Methylbenzylalkohol, 4-Methylbenzylalkohol, 2-Methoxybenzylalkohol, 3-Methoxybenzylalkohol, 4-Methoxybenzylalkohol, 2,3-Dimethylbenzylalkohol, 3,4-Dimethylbenzylalkohol, 2,6-Dimethylbenzylalkohol, 3,5-Dimethylbenzylalkohol, 2,3-Dimethoxybenzylalkohol, 3,4-Dimethoxybenzylalkohol, 3,5-Dimethoxybenzylalkohol, 2-Ethylbenzylalkohol, 3-Ethylbenzylalkohol, 4-Ethylbenzylalkohol, 2,3-Diethylbenzylalkohol, 3,4-Diethylbenzylalkohol, 2,6-Diethylbenzylalkohol, 3,5-Diethylbenzylalkohol, 2-Ethoxybenzylalkohol, 3-Ethoxybenzylalkohol, 4-Ethoxybenzylalkohol, 2-n-Propylbenzylalkohol, 3-n-Propylbenzylalkohol, 4-n-Propylbenzylalkohol, 2,3-Di-n-propylbenzylalkohol, 3,4-Di-n-propylbenzylalkohol, 2,6-Di-n-propylbenzylalkohol, 3,5-Di-n-propylbenzylalkohol, 2-Isopropylbenzylalkohol, 3-Isopropylbenzylalkohol, 4-Isopropylbenzylalkohol, 2-Butylbenzylalkohol, 3-Butylbenzylalkohol, 4-Butylbenzylalkohol, 2-Isobutylbenzylalkohol, 3-Isobutylbenzylalkohol, 4-Isobutylbenzylalkohol, 2-tert.-Butylbenzylalkohol, 3-tert.-Butylbenzylalkohol, 4-tert.-Butylbenzylalkohol, 2,3-Diethoxybenzylalkohol, 3,4-Diethoxybenzylalkohol, 2,6-Diethoxybenzylalkohol, 3,5-Diethoxybenzylalkohol, Benzylalkohol; 2,3,4-Trimethoxybenzylalkohol, 3,4,5-Trimethoxybenzylalkohol, 2,4,6-Trimethoxybenzylalkohol und entsprechende durch die Ethyl-, n-Propyl-, Isopropyl-, n-Butyl-, sek.-Butyl-, Isobutyl-, tert.-Butylgruppe veretherte Trihydroxybenzylalkohole; unsubstituierte oder in vorgenannter Weise substituierte o-, m-oder p-Aminobenzylalkohole, deren Stickstoffatome durch die Methyl-, Ethyl-, n-Propyl-, Isopropyl-, n-Butyl-, sek.-Butyl-, Isobutyl-, tert.-Butylgruppe zweifach substituiert sind; o-, m- und p-Hydroxymethyl-benzylalkohol und entsprechende, in vorgenannter Weise am Kern substituierte Di-(hydroxymethyl)-benzole; unsubstituierte oder in vorgenannter Weise substituierte Phenylethyl-, Phenylpropyl-, Phenylisopropyl-, Phenylbutyl-, Phenyl-sek.-butyl-, Phenyltert.-butyl-, Phenylisobutyl-, Phenylpentyl-, Phenylhexyl-, Phenylisopentylalkohole.

Für die Umsetzung wird Alkohol der allgemeinen Formel II oder Alkohol der allgemeinen Formel II' zusammen mit einem Lösemittel in Dampfform und vorteilhaft im Gemisch mit Inertgas dem Reaktionsraum zugeführt. Als unter Reaktionsbedingungen inerte Lösemittel werden zweckmäßig Tetrahydrofuran, Dioxan, Toluol oder $H_2O$ verwendet. Das Verhältnis Lösemittel zu Alkohol beträgt vorteilhaft 0 bis 1:1. Als Inertgas können sowohl Edelgase, bevorzugt jedoch Stickstoff, Kohlenoxid und/oder Kohlendioxid, verwendet werden. Das Molverhältnis von Inertgas zu Sauerstoff beträgt mindestens 4,4:1, zweckmäßig 4,4 bis 20:1, wobei sich die Angaben bezüglich Inertgas stets auf die Gesamtmenge, d.h. des Inertgasanteils der bevorzugt verwendeten Luft beziehen.

Als oxidierendes Agens lassen sich sowohl der reine Sauerstoff als auch Sauerstoff enthaltende Gase, insbesondere Luft, verwenden. Sauerstoff, in der Regel in Gestalt von Luft und Alkohol, werden zweckmäßig im Molverhältnis von 0,1 bis 0,5, insbesondere 0,15 bis 0,35 Mol Sauerstoff je Mol Alkohol angewandt.

Die Verweilzeit des Gasgemisches im Reaktionsraum beträgt 0,0005 bis 1, bevorzugt 0,001 bis 0,05 Sekunden, Aufgrund der kurzen Verweilzeit werden Nebenreaktionen fast vollständig unterdrückt.

Im erfindungsgemäßen Rohrbündelreaktor werden die Reaktionsrohre mit dem Katalysator beschickt. Die Schichtdicke des Katalysators beträgt zweckmäßig 10 bis 150, vorzugsweise 20 bis 80 mm. Die Katalysatorteilchen werden beispielsweise auf ein Netz aus Silber oder Edelstahl in den vertikal aufgestellten Reaktor geschichtet. Als Katalysatorteilchen eignen sich Teilchen der Korngröße 0,1 bis 5 mm.

Die Standzeit des Katalysators mit konstanter Aktivität und Selektivität beträgt 3 Monate und mehr. Der Katalysator wird zweckmäßigerweise mit 0,2 bis 2 t, insbesondere 0,3 bis 1,5 t Alkohol je $m^2$ Katalysatorquerschnitt und Stunde belastet.

Für die oxidative Dehydrierung leitet man das Gasgemisch aus Alkohol, Luft und Inertgas in den genannten Mengen bei Temperaturen von 300 bis 600 °C, vorzugsweise bei 380 bis 520 °C, durch den Katalysator. Die Reaktion wird im allgemeinen bei Drücken zwischen 0,8 bis 2 bar, vorzugsweise 1,05 bis 1,5 bar, kontinuierlich durchgeführt.

Die aus dem Reaktor austretenden Reaktionsgase werden im Gleichstrom mit dem Produktgemisch oder einem inerten Lösungsmittel oder dem produktenthaltenden Lösungsmittelgemisch rasch auf Raumtemperatur abgekühlt und kondensiert. Als Lösungsmittel eignet sich Wasser oder ein mit Wasser zwei Phasen bildendes Lösungsmittel aus der Verbindungsklasse der Kohlenwasserstoffe, substituierten Kohlenwasserstoffe oder Ether, wie Hexan, Toluol oder Methyl-tert.-butylether. Das Gemisch wird zusammen mit den Reaktionsgasen über eine mit Glasringen gefüllte Säule zum vollständigen Lösen der Produkte im Lösungsmittel geführt.

Durch die Wahl der Quenchflüssigkeit, der Temperatur und der Menge Quenchflüssigkeit können die kondensierbaren Anteile vollständig gewonnen werden.

Die Erfindung wird durch die nachstehenden Beispiele und Vergleichsbeispiele weiter erläutert.

Beispiel 1

Herstellung von 3-Methyl-2-buten-al

Wie in Figur 6 dargestellt, verwendet man eine Anlage mit Zuführungen für Luft (1) und Eduktdampf (2) auf einen Kurzrohrbündelreaktor (3). Die Kontaktrohre sind 60 mm hoch mit Silberkatalysatoren der Korngröße 0,4 bis 0,7 mm gefüllt. Die Kontaktrohre werden von einer Salzschmelze (4) zur Wärmeabfuhr umströmt.

Die heißen Reaktionsgase werden in einem Quench mit anschließender Kolonne (5), der mit der Wasserphase (6) des Produktaustrags beschickt wird, abgekühlt. Über die Leitung (8) bzw. (7) werden die beiden flüssigen Phasen des Austrages zur Aufarbeitung entnommen; über Leitung (9) werden die gasförmigen Anteile entsorgt.

Dem Reaktor werden pro Stunde und Rohr (12 mm ∅) 44 Nl Luft und 113 g 3-Methyl-3-buten-1-ol-dampf zugeführt. Die Temperatur beträgt an der heißesten Stelle 420°C.

Man erhält nach Isomerisierung des 3-Methyl-3-buten-als zu 3-Methyl-2-buten-al 55,8 g 3-Methyl-2-buten-al und 50,9 g nicht umgesetztes 3-Methyl-3-buten-1-ol entsprechend einem Umsatz von 55 % und einer Selektivität von 92 %.

Beispiel 2

Herstellung von 3-Methyl-2-buten-al

Unter Befolgung der Arbeitsweise des Beispiels 1 verwendet man statt des feinen Silberkatalysators eine Silberfraktion mit der Korngröße 1,0 -2,5 mm in einer Schütthöhe von 100 mm. Bei Zufuhr von 44 Nl Luft, 113 g 3-Methyl-3-buten-1-ol-dampf und 21,5 g Wasserdampf pro Stunde und Rohr zum Reaktor erhält man bei einer Maximaltemperatur im Katalysator von 440°C (Salzbadtemperatur 385°C) 520 g nicht umgesetztes 3-Methyl-3-buten-1-ol und 53,6 g 3-Methyl-2-buten-al entsprechend einem Umsatz an 3-Methyl-3-buten-1-ol von 54 % und einer Selektivität von 90 %.

Beispiel 3

Umsetzung von 3-Methyl-2-buten-1-ol zu 3-Methyl-2-buten-al

Man verfährt analog Beispiel 2, setzt jedoch statt 3-Methyl-3-buten-1-ol 3-Methyl-2-buten-1-ol in der gleichen Menge ein.

Man erhält bei einer Reaktionstemperatur von 430°C (Salzbadtemperatur 380°C) 50,2 g nicht umgesetztes 3-Methyl-2-buten-1-ol und 56,6 g 3-Methyl-2-buten-al entsprechend einem Umsatz von 55,6 % an 3-Methyl-2-buten-1-ol und einer Selektivität von 92,3 %.

Beispiel 4

Gemäß dem Beispiel 1 wird in einer Laborapparatur, die nur aus einem Salzbad-gekühlten Rohr besteht, 3-Buten-1-ol zu 3-Buten-al umgesetzt.

Bei Zufuhr von 21 Nl Luft und 56,5 g 3-Buten-1-ol pro Stunde werden bei einer Kontakttemperatur von 420°C (Salztemperatur 380°C) 18,7 g nicht umgesetztes 3-Buten-1-ol und 26,5 g 3-Butenal erhalten. Dies entspricht einem Umsatz von 67 % und einer Selektivität von 72 %.

Beispiel 5

Gemäß dem Beispiel 4 werden 65,3 g 4-Penten-1-ol mit 20,3 Nl Luft pro Stunde bei 440°C (Salztemperatur 390°C) umgesetzt. Man erhält 28,7 g nicht umgesetztes 4-Penten-1-ol und 32,4 g 4-Pentenal, entsprechend einem Umsatz von 66 % und einer Selektivität von 77 %.

Vergleichsbeispiel 1

Herstellung von 3-Methyl-2-butenal im Tiegelreaktor (Labormaßstab)

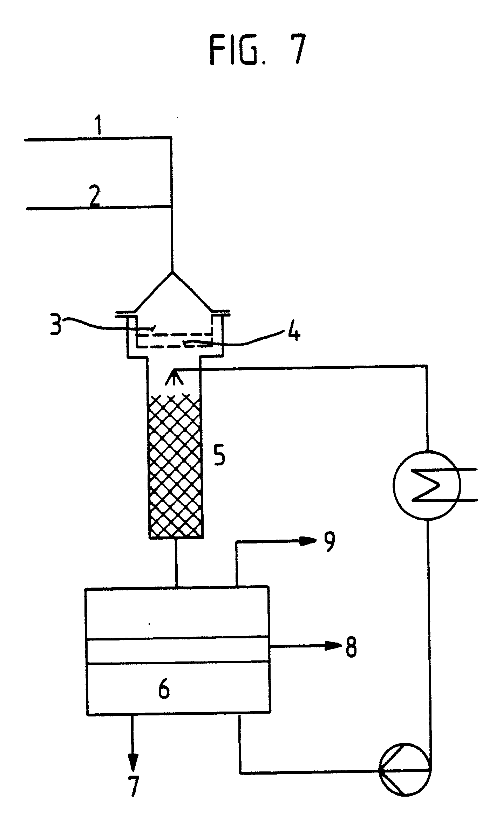

Wie in Figur 7 dargestellt, verwendet man eine Anlage mit Zuführungen für Luft (1) und Eduktdampf (2) auf einen Tiegelreaktor. Der Tiegel hat einen Durchmesser von 20 mm. Der Tiegel enthält eine Silberkatalysatorschicht folgender Zusammensetzung (in Strömungsrichtung gesehen):

| | Schütthöhe (mm) | Korngröße (mm) |
|---|---|---|
| Schicht 1 | 10 | 0,4 - 0,7 |
| Schicht 2 | 11 | 0,7 - 1,0 |

Die den Reaktor verlassenden Reaktionsgase werden wie in Beispiel 1 beschrieben behandelt.

Dem Labortiegelreaktor werden nach Vorheizen von außen pro Stunde 58,5 Nl Luft und 157,1 g 3-Methyl-3-buten-1-ol-dampf zugeführt. Die sich nach Wegnahme der Außenheizung einstellende Temperatur beträgt 495° C. Man erhält nach der üblichen Aufarbeitung 72,3 g nicht umgesetztes 3-Methyl-3-buten-1-ol entsprechend einem Umsatz von 54 % sowie 70,7 g 3-Methyl-2-buten-al. Dies entspricht einer Selektivität von 85,4 %.

Vergleichsbeispiel 2

Herstellung von 3-Methyl-2-butenal im Tiegelreaktor (Produktionsmaßstab)

Man verwendet eine Anlage gemäß Figur 7 mit Zuführung für Luft (1) und Eduktdampf (2) auf einen Tiegelreaktor (3).

Der Tiegel hat einen Durchmesser von 800 mm. Der gelochte Boden ist mit einem Edelstahlnetz belegt. Darauf befindet sich eine Schüttung von Silberkristallen folgender Zusammensetzung:

| | Schütthöhe (mm) | Korngröße (mm) |
|---|---|---|
| Schicht 1 oben | 10 | 0,4 - 0,7 |
| Schicht 2 unten | 11 | 0,7 - 1,0 |

Die den Reaktor verlassenden Reaktionsgase werden wie in Beispiel 1 behandelt.

Dem Tiegelreaktor werden nach Vorheizen mit heißem Inertgas pro Stunde 148 $Nm^3$ Luft und 480 kg 3-Methyl-3-buten-1-ol dampf zugeführt. Die sich dabei einstellende adiabate Temperatur beträgt nach Wegnahme der heißen Inertgase 505° C. Man erhält nach der üblichen Aufarbeitung 202 kg nicht umgesetztes 3-Methyl-3-buten-1-ol entsprechend einem Umsatz von 57,9 % sowie 152 kg 3-Methyl-2-butenal. Dies entspricht einer Selektivität von 56 %.

Vergleichsbeispiel 3

In einem Labortiegelreaktor gemäß Vergleichsbeispiel 1 wird 3-Buten-1-ol zu 3-Butenal umgesetzt. Beim Einsatz von 55,9 Nl Luft und 151 g 3-Buten-1-ol pro Stunde werden bei 510° C Reaktionstemperatur aus dem Austrag 10,6 g nicht umgesetztes 3-Buten-1-ol und 20,5 g 3-Butenal entsprechend einem Umsatz an 3-Buten-1-ol von 93 % und einer Selektivität von 15 % erhalten.

Vergleichsbeispiel 4

Analog dem Vergleichsbeispiel 3 wird 4-Penten-1-ol zu 4-Pentenal umgesetzt.

Bei einer Reaktionstemperatur von 500° C werden aus 60 Nl Luft und 163 g 4-Penten-1-ol pro Stunde 40,6 g nicht umgesetztes 4-Penten-1-ol und 48,9 g 4-Pentenal erhalten. Dies entspricht einem Umsatz von 75,1 % und einer Selektivität von 40,9 %.

## Ansprüche

1. Rohrbündelreaktor zur Durchführung katalytischer organischer Reaktionen in der Gasphase mit zwischen Rohrböden (B) angeordneten Reaktionsrohren (A), dadurch gekennzeichnet, daß die Reaktionsrohre einen Innendurchmesser im Bereich von 0,5 bis 3 cm aufweisen, das Verhältnis Reaktionsrohrlänge zu Innendurchmesser zwischen 2 und 10 liegt und daß die Reaktionsrohre (A) von einem fluiden Wärmeträger in seitlicher Richtung umströmt werden.

2. Rohrbündelreaktor nach Anspruch 1, dadurch gekennzeichnet, daß die Reaktionsrohre (A) eine Länge von 5 bis 20 cm aufweisen.

3. Rohrbündelreaktor nach einem der Ansprüche 1 oder 2, dadurch gekennzeichnet, daß der Rohrinnendurchmesser 1 bis 2 cm beträgt.

4. Rohrbündelreaktor nach einem der Ansprüche 1 bis 3, dadurch gekennzeichnet, daß der fluide Wärmeträger eine Salzschmelze ist, deren Strömungsrichtung im wesentlichen senkrecht zur Längsachse des jeweiligen Reaktionsrohrs verläuft.

5. Verwendung des Rohrbündelreaktors nach einem der Ansprüche 1 bis 4 bei exothermen organischen Reaktionen.

6. Verfahren zur Herstellung aliphatischer, aromatischer oder araliphatischer Ketone und Aldehyde durch oxidative Dehydrierung der entsprechenden Alkohole mit einem Katalysator der Gruppe 1b des Periodensystems der Elemente, dadurch gekennzeichnet, daß man die oxidative Dehydrierung in einem Rohrbündelreaktor nach einem der Ansprüche 1 bis 4 durchführt.

7. Verfahren nach Anspruch 6 zur Herstellung von 3-Alkyl-buten-1-alen der allgemeinen Formel I

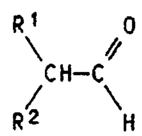

(I)

worin $R^1$ für Wasserstoff steht und $R^2$ den Rest

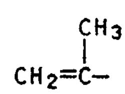

bedeutet oder $R^1$ und $R^2$ zusammen den Rest

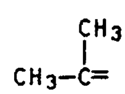

bedeuten,
dadurch gekennzeichnet, daß man 3-Alkyl-buten-1-ole der allgemeinen Formel II

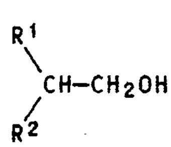

(II)

worin $R^1$ und $R^2$ wie zuvor definiert sind,

mit Sauerstoff bei Temperaturen von 300 bis 600 °C über den Katalysator leitet und die Reaktionsgase nach dem Abkühlen in üblicher Weise aufarbeitet.

## Claims

1. A tube bundle reactor for carrying out catalytic organic reactions in the gas phase using reaction tubes (A) arranged between tubesheets (B), wherein the reaction tubes have an inside diameter of from 0.5 to 3 cm, the ratio of reaction tube length to inside diameter ranges from 2 to 10 and the reaction tubes (A) are surrounded by a fluid heat transport medium flowing in the crosswise direction.

2. A tube bundle reactor as claimed in claim 1, wherein the reaction tubes (A) have a length of from 5 to 20 cm.

3. A tube bundle reactor as claimed in claim 1 or 2, wherein the tube inside diameter ranges from 1 to 2 cm.

4. A tube bundle reactor as claimed in any one of claims 1 to 3, wherein the fluid heat transport medium is a molten salt whose direction of flow is substantially at right angles to the longitudinal axis of the particular reaction tube.

5. Use of the tube bundle reactor as claimed in any one of claims 1 to 4, in exothermic organic reactions.

6. A process for preparing an aliphatic, aromatic or araliphatic ketone or aldehyde by oxidative dehydrogenation of the corresponding alcohol with a catalyst of group 1b of the periodic table, which comprises carrying out the oxidative dehydrogenation in a tube bundle reactor as claimed in any one of claims 1 to 4.

7. A process as claimed in claim 6 for preparing a 3-alkylbuten-1-al of the formula I

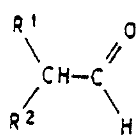

(I)

where $R^1$ is hydrogen and $R^2$ is

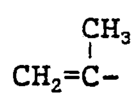

or $R^1$ and $R^2$ are together

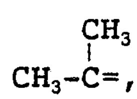

wherein a 3-alkylbuten-1-ol of the formula II

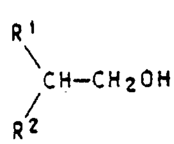

(II)

where $R^1$ and $R^2$ are as defined above, is passed together with oxygen over a catalyst at from 300 to 600 °C and, after cooling down, the reaction gas is worked up in a conventional manner.

## Revendications

1. Réacteur à faisceau tubulaire pour l'exécution de réactions organiques catalytiques en phase gazeuse, comportant des tubes de réaction (A) disposés entre des plaques tubulaires (B) caractérisé en ce que les tubes de réaction présentent un diamètre intérieur compris entre 0,5 et 3 cm, en ce que le rapport de la longueur au diamètre intérieur des tubes de réaction se situe entre 2 et 10, et en ce que les tubes de réaction (A) sont balayés en direction latérale par un courant de caloporteur fluide.

2. Réacteur à faisceau tubulaire selon la revendication 1, caractérisé en ce que les tubes de réaction (A) présentent une longueur de 5 à 20 cm.

3. Réacteur à faisceau tubulaire selon la revendication 1 ou 2, caractérisé en ce que le diamètre intérieur des tubes mesure de 1 à 2 cm.

4. Réacteur à faisceau tubulaire selon l'une quelconque des revendications 1 à 3, caractérisé en ce que le caloporteur fluide est une masse fondue de sel dont la direction d'écoulement est pratiquement perpendiculaire à l'axe longitudinal de chaque tube de réaction.

5. Utilisation du réacteur à faisceau tubulaire selon l'une quelconque des revendications 1 à 4 dans des réactions organiques exothermiques.

6. Procédé de préparation de cétones et d'aldéhydes aliphatiques, aromatiques ou araliphatiques par déshydrogénation par voie d'oxydation des alcools correspondants en présence d'un catalyseur du groupe Ib de la classification périodique des éléments, caractérisé en ce qu'on effectue la déshydrogénation par voie d'oxydation dans un réacteur à faisceau tubulaire selon l'une quelconque des revendications 1 à 4.

7. Procédé selon la revendication 6 pour la préparation de 3-alkylbutène-1-als de formule générale I

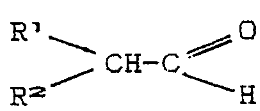

(I) dans laquelle $R^1$ est mis pour un atome d'hydrogène et $R^2$ représente le reste

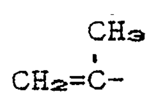

ou $R^1$ et $R^2$ représentent ensemble le reste

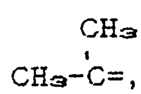

caractérisé en ce qu'on envoie des 3-alkylbutène-1-ols de formule générale II

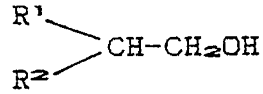

(II) dans laquelle $R^1$ et $R^2$ sont tels que définis précédemment, avec de 1'oxygène à une température

de 300 a 600 °C sur le catalyseur et, après refroidissement, on soumet les gaz réactionnels à un traitement ultérieur usuel.

FIG. 1

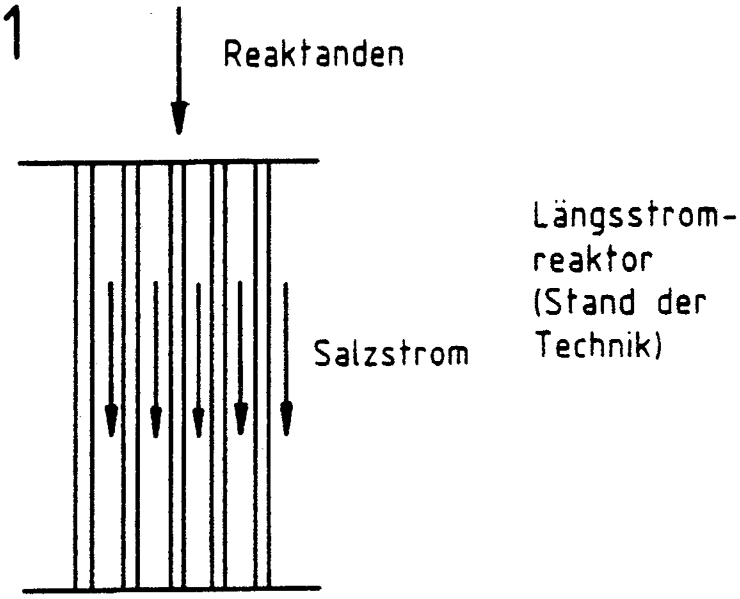
Reaktanden
Salzstrom
Längsstrom-
reaktor
(Stand der
Technik)

FIG. 2

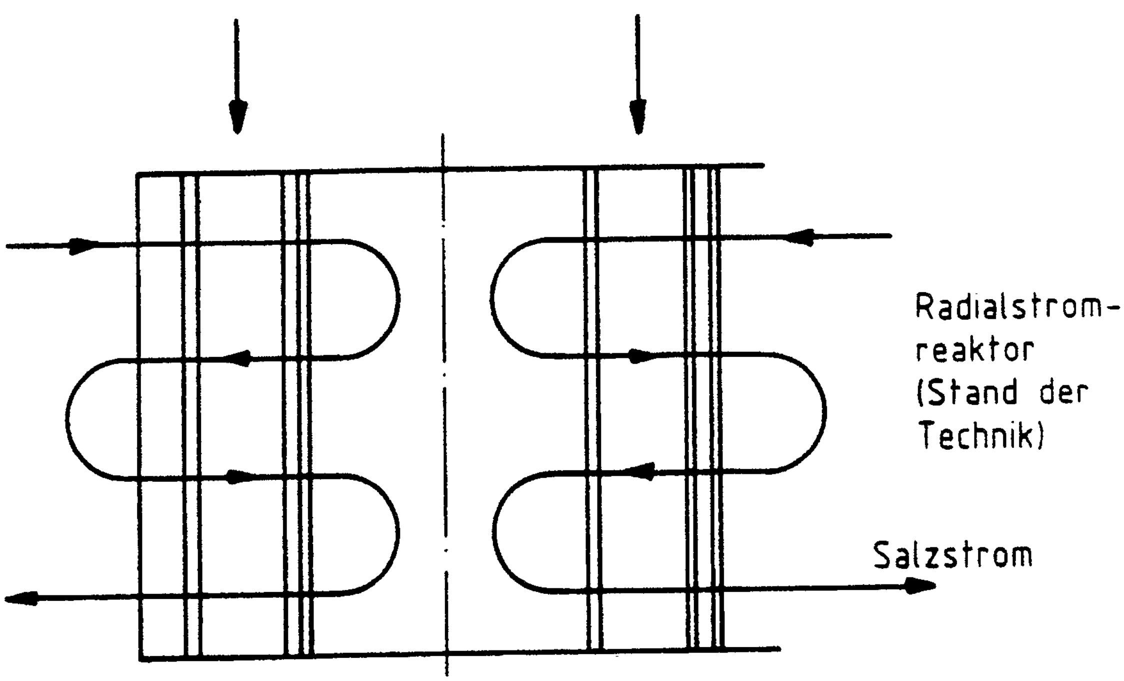
Radialstrom-
reaktor
(Stand der
Technik)
Salzstrom

FIG. 3

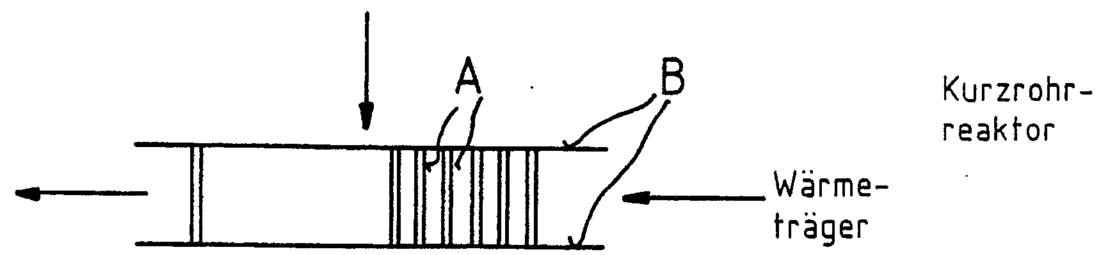
A
B
Kurzrohr-
reaktor
Wärme-
träger

FIG. 4

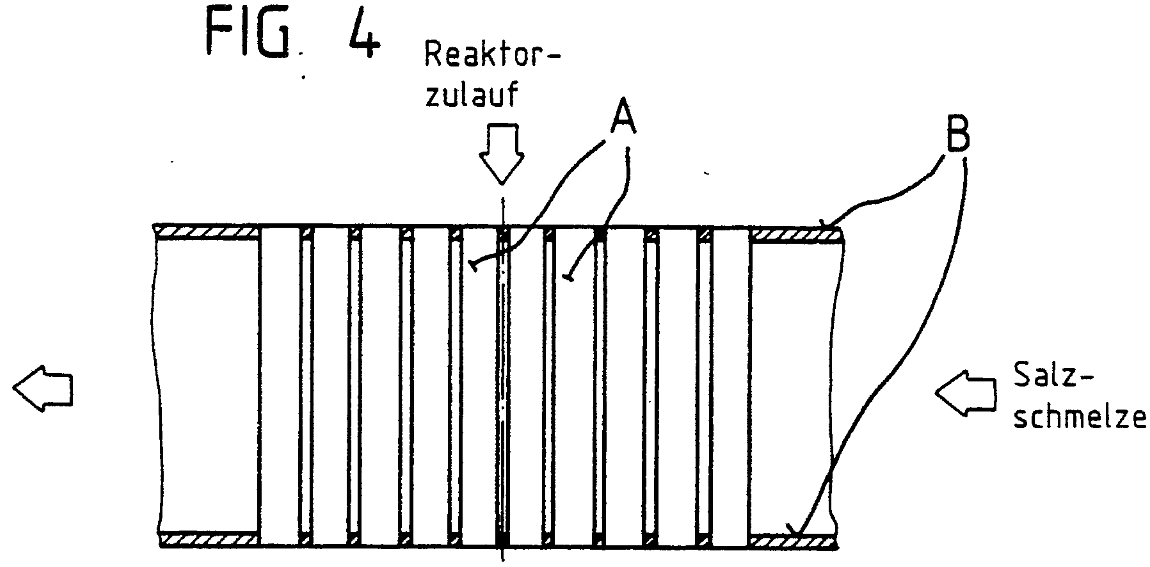
Reaktor-
zulauf
A
B
Salz-
schmelze

FIG. 5

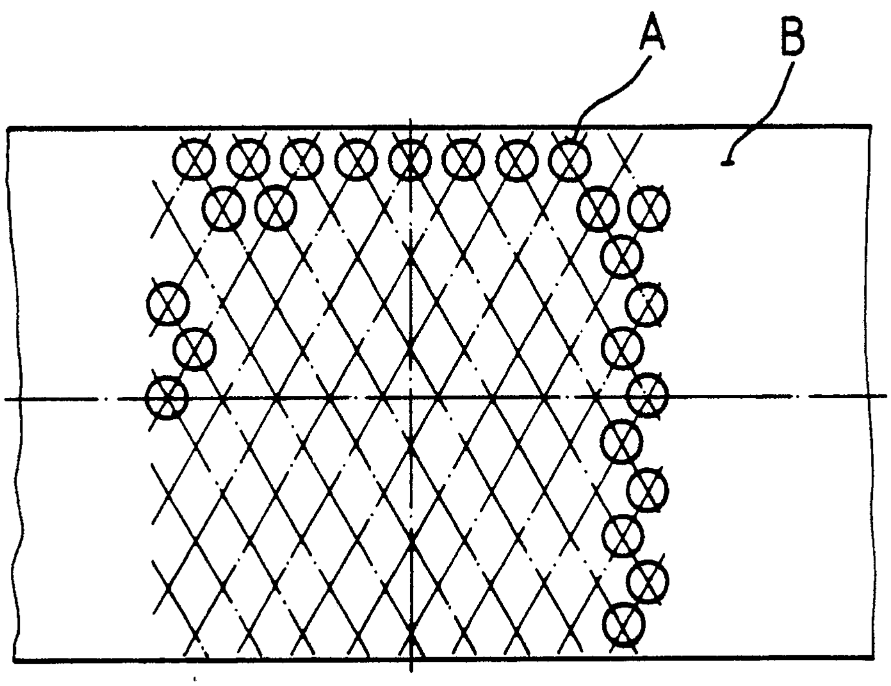

FIG. 6

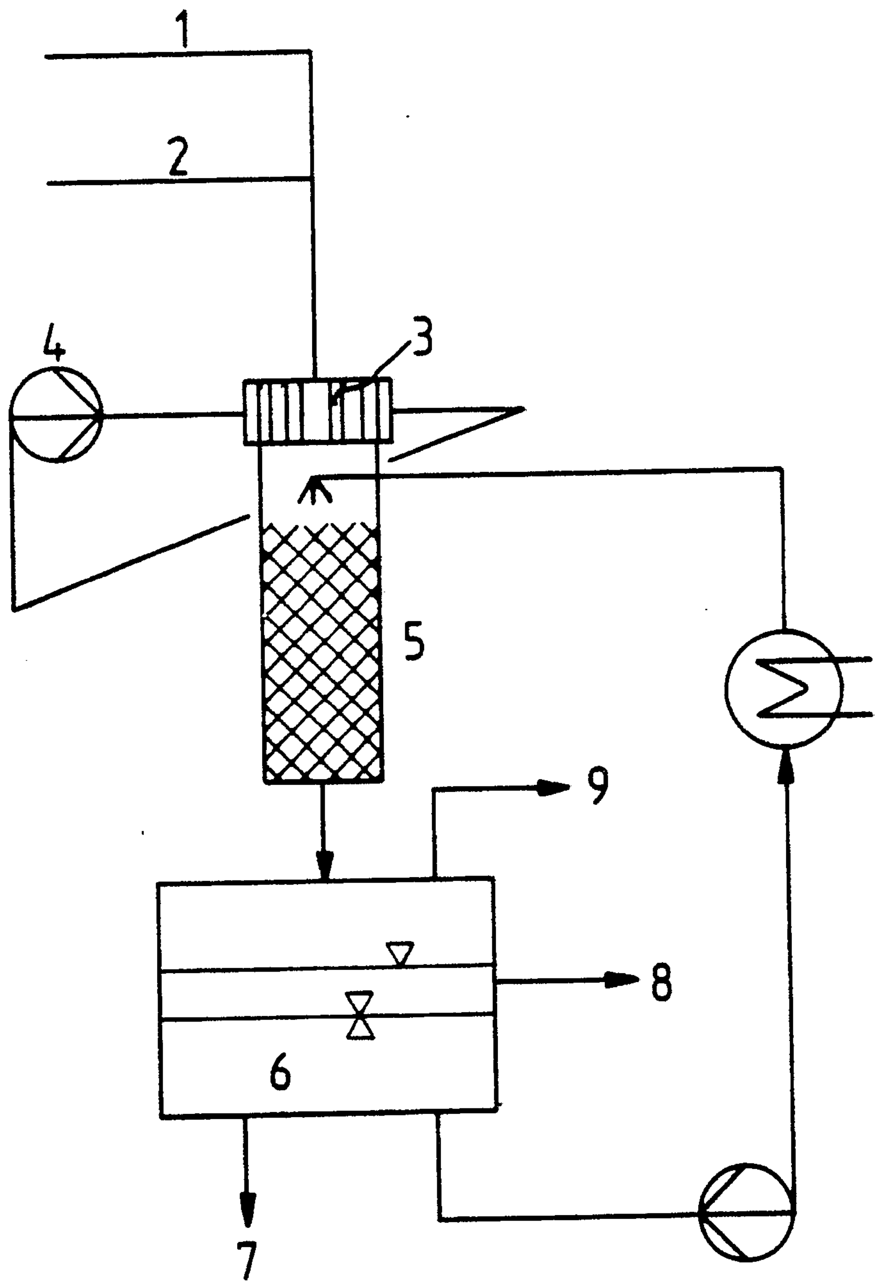

FIG. 7